# EUROPEAN PATENT SPECIFICATION

(11) **EP 0 401 232 B1**
(45) Date of publication and mention of the grant of the patent: **21.07.1993**
(21) Application number: 89901847.7
(22) Date of filing: 13.01.1989
(51) Int. Cl.: A61L 2/04, A61K 35/16, A61K 31/725

(54) **A PROCESS FOR PASTEURIZATION OF AQUEOUS SOLUTIONS OF FACTOR VIII**
VERFAHREN ZUR PASTEURISIERUNG VON WÄSSRIGEN LÖSUNGEN VON FAKTOR VIII
PROCEDE DE PASTEURISATION DE SOLUTIONS AQUEUSES DE FACTEUR VIII

(30) Priority: 15.01.1988 DK 182/88
(43) Date of publication of application: 12.12.1990
(73) Proprietor: NOVO NORDISK A/S, 2880 Bagsvaerd (DK)
(72) Inventor: KIHL, Jesper, DK-2800 Lyngby (DK); NYRUP, Allan, DK-2800 Lyngby (DK); NORDFANG, Ole, DK-3400 Hillerod (DK)
(86) International application number: DK8900003
(87) International publication number: WO8906547

(56) References cited:
- EP-A- 0 137 428
- GB-A- 2 172 000
- US-A- 4 297 344
- US-A- 4 388 232
- US-A- 4 562 072
- DIALOG INFORMATION SERVICES, file 155, medline 66-89, Dialog accession no. 06539236, Smit Sibinga CT: "Double cryoprecipitated factor VIII condentrate from heparinised plasma and its heat treatment", Smit Sibinga CT, Blut Mar 1988, 56(3) p111-6

## Description

The present invention concerns a process for pasteurization of aqueous solutions of of coagulation Factor VIII, in particular human Factor VIII. The object of the invention is to remove or inactivate infectious virus which might be present in preparations containing the coagulation Factor VIII. A particular object of the invention is to carry out heat treatment of aqueous solutions of Factor VIII under conditions where any present viruses are inactivated with certainty, while obtaining high recovery of the therapeutic protein human coagulation Factor VIII.

The human coagulation Factor VIII (hereinafter called Factor VIII), also called Antihemophilic Factor (AHF), forms part of the coagulation system of the blood. Factor VIII must be present for coagulation to take place. Humans having completely or partly lost the ability to synthesize Factor VIII suffer from the disease Hemophilia A, which is a congenital disease. The disease is characterized by frequent bleedings, in particular in the joints and in the musculature.

It is thus necessary to substitute humans having Factor VIII deficiency with preparations containing Factor VIII. Such a substitution therapy of Hemophilia A patients was initiated in Europe in 1965. This substitution therapy has resulted in a considerable increase in the average duration of life of Hemophilia A patients. Thus, the average duration of life for Hemophilia A patients in Sweden has increased from about 16 years in 1948 to about 56 years in 1978. Furthermore, the substitution therapy has caused a reduction in the number of disabling bleedings in Hemophilia A patients and thus improved the patients' ability to earn their living and their conditions of life.

Preparations containing Factor VIII are prepared from human blood or blood plasma. Various processes for this production are known. For all these, the initial process is equivalent. Blood from donor may be tapped in a bag system containing anticoagulant. The anticoagulant may be citrate phosphate dextrose (CPD), acid citrate dextrose (ACD) or heparin. The blood plasma is separated from the other components by centrifugation. This separation may also take place in the actual tapping of donor by so-called plasma pheresis. The blood plasma is stored in a frozen state. The frozen blood plasma is thawed, and so-called cryoprecipitation is carried out. The resulting cryoprecipitate contains Factor VIII and forms the starting point for the further production.

This may be performed in a known manner using precipitation techniques for removing undesirable protein material. Precipitation of this protein material may for example take place by addition of ethanol (Mattock et al., European Patent Application No. 0 062 456, 1982), of polyethylene glycol (Sarno et al., European Patent Application No. 0 221 556, 1987) or glycine (Mitra et al., European Patent Application No. 0 126 789, 1984). Other known processes for removing undesirable protein material from solutions containing Factor VIII comprise using chromatographic techniques (for example as described by Chavin et al., European Patent Application No. 0 104 356, 1984).

It is moreover known that the use of blood plasma produced with heparin as anticoagulant, so-called heparinized blood plasma, can increase the recovery of Factor VIII in the cryoprecipitate (Rock et al., European Patent Application No. 0 053 046, 1982). Heparin is a group of sulphated polysaccharides having a molecular weight from 5000 to 40000, typically from 15000 to 25000, and has an anticoagulating effect in blood plasma. Even though heparin can increase the recovery of Factor VIII, heparin is not extensively used as anticoagulant because this prevents using a number of the other components of the blood for therapeutic purposes.

Heparin has an anticoagulating effect in blood or blood plasma. Heparin per se partly inhibits thrombin, partly acts as a catalyzer in the formation of a thrombin - antithrombin III complex. Thrombin is one of the proteins which can cleave the Factor VIII molecule in fragments. Factor VIII is an important coagulation Factor in the coagulation system of the blood. Activation of Factor VIII causes cleavage of the Factor VIII molecule in fragments. This activation is caused either by activated Factor X activated protein C or thrombin, of which the latter is the most important one. Thus, addition of heparin affects the natural coagulation system and consequently stabilises Factor VIII.

Heparin does not affect the content of free calcium ions in blood or blood plasma containing Factor VIII. This is in contrast to citrate or citrate-containing anticoagulants such as citrate phosphate dextrose (CPD), where the present citrate acts as a chelate former and forms a complex with calcium. Since the presence of calcium is of importance for the stability of Factor VIII, the use of heparin as an anticoagulant may be instrumental in improving the stability of Factor VIII.

Stabilization of Factor VIII with heparin in heparinized blood plasma thus takes place partly by inhibition of thrombin, which degrades Factor VIII, and partly by not changing the presence of calcium ions.

Factor VIII preparations may be prepared from heparinized plasma having a high recovery of Factor VIII activity in the cyroprecipitate (Smit-Sibinga et al., Lancet, ii, 449, 1981). The reason is partly that heparin stabilizes Factor VIII in the plasma, partly that heparin increases precipitation of Factor VIII by the cryoprecipitation.

For a higher recovery of Factor VIII in the cryoprecipitate to be obtained, it is important that the plasma is heparinized during tapping of the donor. Later addition of heparin to blood plasma produced with citrate-containing anticoagulant does not result in an increased recovery of Factor VIII in the cryoprocipitate (Mikaelsson et al., Blood, Vol. 62, No. 5, 1983, p. 1006). Likewise, addition of heparin during the production of preparations containing Factor VIII after or during redissolution of the cryoprecipitate does not improve the recovery of Factor VIII. Thus, the effect of heparin depends upon addition of heparin in connection with tapping of the donor. On the other hand, addition of both heparin and calcium to citrate-containing blood plasma or fractions may result in an increased recovery of Factor VIII.

A serious problem in the therapeutic use of preparations or fractions produced from human blood or blood plasma is that this blood or blood plasma may contain infectious virus. Examples of such infectious virus are Hepatitis B Virus, Hepatitis non-A-non-B Virus and Human Immunodeficiency Virus. Transmission of infectious virus is known not only from the use of preparations containing Factor VIII, but generally from the use of preparations produced from human blood or blood plasma. Examples of such preparations are coagulation Factor IX, coagulation Factor XIII, immunoglobulin, antithrombin III.

Known are various processes for removing infectious virus from therapeutic preparations or solutions containing these produced from human blood or blood. Such a known process comprises heating for 10 hours at 60 °C of aqueous solutions containing therapeutic protein, which provides certainty against transfer of infectious virus when using preparations containing these plasma proteins. To avoid denaturation or other damage to the therapeutically active protein in the aqueous solution it is necessary, however, to add stabilizers during the heat treatment.

Thus, salts of fatty acids or amino acid are added as stabilizers in a heat treatment for 10 hours at 60 °C of solutions containing human albumin or protein concentrate (Gellis et al, J. Clin. Invest., 27, 239, (1948)). The use of this process for treating human albumin or protein concentrate has entailed that no infectious virus has been transferred using these preparations since this process was introduced in 1948. In this process, there are no or only insignificant losses of the therapeutic plasma protein albumin.

Solutions containing plasminogen may be stabilized with the amino acid lysin under a heat treatment at 60 °C for 10 hours (Baumgarten et al, USA Patent No. 3 227 626, 1966). According to the patent this process destroys present hepatitis virus.

Solutions containing the coagulation Factor XIII may be heated to 60 °C for 10 hours in the presence of amino acid, monosaccharide or sugar alcohol (Fukushima, Patent No. Sho 51-134878, 1976). A loss of Factor XIII activity of about 50% has been reported for this process.

Further, solutions containing the proteinase inhibitor Antithrombin III may be stabilized with salts of citric acids before heating for 10 hours at 60 °C (Holleman et al., Thromb. & Haemo., 38, 201, (1977)). The loss of the therapeutic protein Antithrombin III amounts to about 30% in this process.

Various processes for stabilizing Factor VIII by heat treatment in aqueous solutions are known.

A process for stabilizing Factor VIII by heat treatment for 10 hours at 60 °C with a polyol has been described (US Patent No. 4 440 697, 1984). This process comprises adding for example saccharose, optionally together with the amino acid glycine, in an amount so that after addition the solution contains 62% w/w saccharose or is saturated with saccharose.

A similar process for stabilizing Factor VIII likewise by heat treatment for 10 hours at 60 °C has been described using sugar alcohol or disaccharide (European Patent Application No. 0 117 064, 1984). This process comprises adding for example 66% w/w saccharose or sorbitol as a stabilizer during the heat treatment.

A process using a combination of either a neutral amino acid, a saccharide or a sugar alcohol and the salt of a carboxylic acid having from 3 to 10 carbon atoms for stabilizing Factor VIII by heat treatment at 60 °C for 10 hours is known (European Patent Application No. 0 077 879, 1983). Human albumin may be added as a further stabilizer. This process comprises adding for example 25% w/v glycine and 20% w/v sodium caprylate as a stabilizer.

Further, known is a process for heat treatment of Factor VIII for 10 hours at 60 °C with simultaneous reduction of a contaminating fibrinogen content (DE Offenlegungsschrift No. 29 16 711, 1980). The stabilizer is a saccharide or sugar alcohol in combination with a high concentration of amino acid. The present amino acid causes the fibrinogen in the Factor VIII solution to be precipitated. This process comprises adding for example 50% w/w saccharose and 2 M glycine to the Factor VIII solution during the heat treatment.

Finally, it is known from UK 2 172 000 to perform heat treatment for 10 hours at 60 °C of a solution containing, in addition to Factor VIII, residual amounts of heparin or other sulfated polysaccharide which has not been removed completely in the previous purification steps. In these previous tests, the presence of such residual amounts of heparin were found to have no influence on the yield or recovery of Factor VIII after the heat treatment (see p. 4, 1. 6-7 in the description of UK 2 172 000).

It is characteristic of the previously known processes for stabilizing Factor VIII in aqueous solutions with heat treatment at 60 °C for 10 hours that the recovery of Factor VIII after the heat treatment is low. Thus, the examples in the patent specifications mentioned above report recovery of Factor VIII after heat treatment for 10 hours at 60 °C as stated in the following table.

| Description of process | Per cent recovery F VIII activity |
|---|---|
| US Pat. No. 4 440 679, example 1 A | 63 |
| US Pat. No. 4 440 679, example 1 B | 61 |
| EP Appl. No. 0 077 870, table 1 | 40 - 70 |
| EP Appl. No. 0 117 064, example 1 | 58 |
| EP Appl. No. 0 117 064, example 2 | 56 |
| EP Appl. No. 0 117 064, example 3 | 58 |
| EP Appl. No. 0 117 064, example 4 | 57 |
| DE Off. No. 29 16 711, example 2 | 52 |

The low recovery of Factor VIII in the previously known processes constitutes a severe drawback of these. Since preparations of fractions containing Factor VIII are produced from human blood or blood plasma originating from donors, this material is a highly limited raw material.

Thus, in Denmark about 20 million IU of Factor VIII (1987) are used per year, corresponding to tapping of about 400,000 donors. 1 IU (international unit) of Factor VIII corresponds to the Factor VIII activity of 1 ml of normal plasma. At the same time, the costs of producing these preparations are very high, and the recovery of Factor VIII activity low, which causes Factor VIII to be a very costly protein. Thus, the price of 1 IU of Factor VIII in a highly purified Factor VIII preparation is about Dkr. 4 in Denmark. The annual consumption of Factor VIII in Denmark corresponds to about Dkr. 80 million. It is thus of great importance that the recovery of Factor VIII is high.

The present invention concerns a process of the type defined in the introductory portion of claim 1 and is characterized by the subject-matter defined in the characterizing portion of the claim. This process prevents transmission of infectious virus when using the preparation and also gives a high recovery of Factor VIII.

The process comprises adding to the solution of Factor VIII heparin as a stabilizer in combination with a saccharide or sugar alcohol, and the heat treatment is carried out in a manner known per se, preferably for 10 hours at 60 °C. This provides a surprisingly improved recovery of Factor VIII.

When adding heparin as a stabilizer in combination with a saccharide or sugar alcohol in a heat treatment for 10 hours at 60 °C of an aqueous solution containing citrate and Factor VIII, it is only the effect of inhibition of thrombin which is important. This effect may be intensified by bonding heparin to von Willebrand Factor. Owing to the present citrate there is no increase in the calcium level with consequent stabilization of Factor VIII.

The effect of adding heparin in a heat treatment may thus be ascribed to an impact on the natural coagulation system with consequent stabilization of Factor VIII.

This is in contrast to the previously known additions in a heat treatment for 10 hours at 60 °C of an aqueous solution containing Factor VIII. Saccharides, sugar alcohol, amino acids or salts of organic acids have a stabilizing effect in that these stabilizers influence the hydration degree or charge difference of the protein.

The process in the heat treatment for 10 hours at 60 °C of an aqueous solution containing Factor VIII with heparin as a stabilizer in combination with a saccharide or sugar alcohol may be performed on any fraction or preparations containing Factor VIII.

A saccharide or sugar alcohol is added to citrate solutions containing Factor VIII. Addition of saccharose is a preferred embodiment. Stabilization of Factor VIII increases with increased addition of saccharide or or sugar alcohol. Thus, from 20 w/w % to saturation may be addead. The preferred embodiment is addition of saccharose corresponding to 60 w/w %. Saturation of a solution containing Factor VIII with saccharide or sugar alcohol causes partly handling difficulties owing to the high viscosity, partly stabilization of the infectious virus.

Heparin may be added before or after addition of saccharide or sugar alcohol corresponding to from 1.0 to 4.0 IU (international units) of heparin per g of solution after addition of saccharide or sugar alcohol. Addition corresponding to from 1.5 to 2.5 IU of heparin per g of solution is a preferred embodiment.

Addition of too large amounts of heparin causes precipitation of Factor VIII. This precipitation may be detected by measuring the cloudiness of the solution. It has thus been found that an increase in the cloudiness of the solution may be detected by addition of 4.0 IU of heparin per g of solution. It applies to precipitation of proteins that chemical and physical conditions in the solution, such as for example protein concentration, protein composition, conductivity, pH or salt concentration, influence the degree of precipitation. Changes in for example protein concentration or protein composition may thus cause changes in the heparin concentration which gives the highest recovery of Factor VIII without the protein solution beginning to precipitate.

To obtain a maximum yield, the pH value must be adjusted in the solution to a physiological level. Thus, the pH value should be between 6.0 and 7.0 with from 6.2 to 7.0 as a preferred process.

Addition of CaCl₂ in limited amounts may cause stabilization of Factor VIII during the heat treatment. In the present process, from 0 to 150 mmols of CaCl₂ per g of ready solution are used.

Removal of saccharide or sugar alcohol from the solution containing Factor VIII after the heat treatment can take place by dialysis or precipitation of Factor VIII from the solution.

Determination of Factor VIII activity can be performed either by a 2-step assay or a 1-step assay. It is known that 1-step and 2-step assays may result in various determinations of the Factor VIII activity in a given sample. It is moreover known that repeated measurements with the same assay on the same sample may give rise to great variations in the determination of Factor VIII activity. It is also known that the results of the determination of Factor VIII activity can depend upon other present proteins or other components in the given sample.

Determination of Factor VIII activity in a 2-step assay is performed by means of a chromogenic substrate method (KABI Coatest Factor VIII), the original method in test tubes at 37 °C having been modified for execution in microtiter plate with reduced reagent use at room temperature. 50 microlitres of sample or standard are used, which, following mixing with 50 microlitres of phospholipid, Factor IXa and Factor X, are warmed for 10 minutes. After addition of 25 microliters of 25 mM CaCl₂, incubation is effected for 15 minutes, and then 50 microlitres of substrate are added.

After an additional 20 minutes' incubation the reaction is stopped by addition of 50 microlitres of 1 M citric acid. The colour development is read at 405 nm with reference at 492 nm.

Determination of Factor VIII activity in 1-step assay is performed by means of the APTT method (Activated Partial Thromboplastin Time). 100 microlitres of sample or standard are pipetted in the cuvettes, and then 100 microlitres of deficient plasma are added (Factor VIII deficient plasma, General Diagnostic). 100 microlitres of APTT-reagent (General Diagnostic) are added, and the solution is thermostated at 37 °C for 5 minutes. After addition of 100 microlitres of 0.03 M CaCl₂, the time until coagulation of the solution is measured.

For quantitative determinations, calibration curves are prepared, based on dilution series of an internal standard calibrated against WHO standard (3rd Int. Standard of F VIII, Human Plasma, 3.9 IU/ml). 2-step assay, as stated here, has a lower detection limit (about 10 times) than 1-step assay. In connection with addition of heparin it is an advantage to use 2-step assay since any heparin effect on the assay can more easily be destroyed by dilution.

Determination of cloudiness is performed by measuring the extinction at 400 nm.

Thus, tests have shown that addition of heparin corresponding to a concentration of from 1.0 to 4.0 IU of heparin per g of solution containing Factor VIII stabilized with a saccharide or sugar alcohol during the heat treatment results in an increase in Factor VIII recovery.

The process of the invention is illustrated by the following examples.

### Example 1

14 ml of citrate solution (0.53 g of sodium citrate/litre) containing 1680 IU Factor VIII) are distributed in 7 tubes so that each tube contains 2 ml of solution. To each tube are added 3 g of saccharose which is caused to dissolve. Heparin solution (500 IU/ml) is additionally added to each tube so that the individual tube contains a heparin amount as stated in the table below, and about 48 IU of Factor VIII of ready solution. The tubes are sealed and heat treated for 10 hours at 60 °C. Factor VII is determined by 1-step assay, and the cloudiness of the solution is determined as the extinction at 400 nm.

| IU heparin added per g solution | Per cent recovery Factor VIII activity | E₄₀₀ |
|---|---|---|
| 0.0 | 70 | 0.35 |
| 0.3 | 70 | 0.34 |
| 0.5 | 71 | 0.35 |
| 1.0 | 87 | 0.35 |
| 2.0 | 91 | 0.37 |
| 4.0 | 93 | 0.40 |
| 8.0 | 85 | 0.63 |

### Example 2

20 ml of citrate solution (0.53 g of sodium citrate/litre) containing 6000 IU Factor VIII are distributed in 10 tubes, so that each tube contains 2 ml of solution. To each tube are added 3 g of saccharose which is caused to dissolve. Heparin solution (500 IU/ml) is additionally added to each tube, so that the individual tube contains a heparin amount as stated in the table below, and about 120 IU of Factor VIII per g of ready solution. The tubes are sealed and heat treated for 10 hours at 60 °C. Factor VIII is determined by 2-step assay.

| IU heparin added per g solution | Per cent recovery of Factor VIII activity |
|---|---|
| 0.0 | 66 |
| 1.0 | 86 |
| 1.5 | 81 |
| 2.0 | 91 |
| 2.5 | 87 |

### Example 3

8 ml of citrate solution (0.53 g of sodium citrate/litre) containing about 5000 IU of Factor VIII are distributed in 4 tubes, so that each tube contains 2 ml of solution. To each tube are added 3 g of saccharose which is caused to dissolve. Heparin solution (500 IU/ml) is additionally added to 2 tubes, so that the tube contains a heparin amount of 2.0 IU per g of solution. The tubes are sealed and heat treated for 10 hours at 60 °C. The test is repeated on different sample material, a total of 5 times. The Factor VIII content varied in these tests from 220 to 260 IU per g of ready solution. Factor VIII is determined by 2-step assay.

| Test | IU heparin per g solution | Per cent recovery of Factor VIII activity | Per cent improvement |
|---|---|---|---|
| 1 | 0 | 40 | |
| | 0 | 40 | |
| | Average | 42 | |
| | 2 | 71 | |
| | 2 | 81 | |
| | Average | 76 | |
| 2 | 0 | 51 | |
| | 0 | 55 | |
| | Average | 53 | 81 |
| | 2 | 86 | |
| | 2 | 87 | |
| | Average | 87 | 63 |
| 3 | 0 | 55 | |
| | 0 | 60 | |
| | Average | 58 | |
| | 2 | 85 | |
| | 2 | 89 | |
| | Average | 87 | 51 |
| 4 | 0 | 71 | |
| | 0 | 71 | |
| | Average | 71 | |
| | 2 | 83 | |
| | 2 | 84 | |
| | Average | 84 | 18 |
| 5 | 0 | 59 | |
| | 0 | 66 | |
| | Average | 63 | |
| | 2 | 84 | |
| | 2 | 85 | |
| | Average | 85 | 35 |
| Average improvement for all tests is 50%. | | | |

### Example 4

2 ml of citrate solution (0.53 g of sodium citrate/litre) containing 600 IU of Factor VIII are mixed with 3 g of saccharose which is caused to dissolve. Heparin solution (500 IU/ml) and calcium chloride (10⁻² M) are additionally added, so that the tube contains a heparin amount of 2.0 IU, 100 mmols calcium chloride and 120 IU Factor VIII per g of solution. The tube is sealed and heat treated for 10 hours at 60 °C. Factor VIII is determined by 2-step assay. Recovery of Factor VIII was 88 %.

## Claims

1. A process for pasteurization of aqueous solutions of Factor VIII by heating the solution to a temperature of 55-65 °C for a period of time of 5-15 hours in the presence of a saccharide or a sugar alcohol, **characterized** by using a purified citrate containing solution of Factor VIII to which is further added 1-4 IU of heparin per g of ready solution followed by heat treatment.

2. A process according to claim 1, **characterized** in that the added heparin amount constitutes 1.5-2.5 IU per g of ready solution.

3. A process according to claim 1 or 2, **characterized** in that the pH value is maintained at 6.2-7.0 during the heat treatment.

4. A process according to any of claims 1-3, **characterized** in that the Factor VIII content is maintained between 10 and 300 IU per g of ready solution.

5. A process according to any of chaims 1-4, **characterized** in that the amount of saccharide or sugar alcohol constitutes 50-65% w/w calculated on the ready solution.

6. A process according to claim 5, **characterized** in that the saccharide is saccharose.

7. A process according to any of claims 1-6, **characterized** by performing the heat treatment in the presence of CaCl₂ in an amount not exceeding 150 mmols per g of ready solution.

## Patentansprüche

1. Verfahren zum Pateurisieren von wässrigen Lösungen mit Faktor VIII durch Erhitzen der Lösung auf eine Temperatur von 55 - 65°C während eines Zeitraums von 5 - 15 Stunden in Gegenwart eines Saccharids oder eines Zuckeralkohols, gekennzeichnet durch Verwenden eines gereinigten Citrats, das eine Lösung mit Faktor VIII enthält, dem ferner 1 - 4 IU Heparin/g an fertiger Lösung zugesetzt wird mit nachfolgender Wärmebehandlung.

2. Verfahren nach Anspruch 1, dadurch gekennzeichnet, dass die zugesetzte Menge Heparin 1.5 bis 2.5 IU an fertiger Lösung beträgt.

3. Verfahren nach Anspruch 1 oder 2, dadurch gekennzeichnet, dass der pH-Wert während der Wärmebehandlung 6.2 - 7.0 beträgt.

4. Verfahren nach einem der Ansprüche 1 bis 3, dadurch gekennzeichnet, dass der Faktor VIII-Gehalt zwischen 10 und 300 IU/g an fertiger Lösung gehalten wird.

5. Verfahren nach einem der Ansprüche 1 bis 4, dadurch gekennzeichnet, dass die Menge an Saccharid oder Zuckeralkohol 50 - 65% w/w ausmacht, berechnet auf der Grundlage der fertigen Lösung.

6. Verfahren nach Anspruch 5, dadurch gekennzeichnet, dass das Saccharid Saccharose ist.

7. Verfahren nach einem der Ansprüche 1 bis 6, gekennzeichnet durch Durchführen der Wärmebehandlung in Gegenwart von CaCl₂ in einer Menge, die 150 mMol/g an fertiger Lösung nicht übersteigt.

## Revendications

1. Un procédé de pasteurisation de solutions aqueuses de facteur VIII par chauffage de la solution à une température de 55 à 65°C pendant une période de 5 à 15 heures, en présence d'un saccharide ou d'un polyalcool glucidique, caractérisé par l'utilisation d'une solution purifiée, contenant du citrate, de facteur VIII à laquelle on a de plus ajouté 1 à 4 UI d'héparine par g de solution prête, puis traitement thermique.

2. Un procédé selon la revendication 1, caractérisé en ce que la quantité d'héparine ajoutée est de 1,5 à 2,5 UI par g de solution prête.

3. Un procédé selon la revendication 1 ou 2, caractérisé en ce que le pH est maintenu entre 6,2 et 7,0 pendant le traitement thermique.

4. Un procédé selon l'une quelconque des revendications 1 à 3, caractérisé en ce que la teneur en facteur VIII est maintenue entre 10 et 300 UI/g de solution prête.

5. Un procédé selon l'une quelconque des revendications 1 à 4, caractérisé en ce que la quantité de saccharide ou de polyalcool glucidique est de 50 à 65 % p/p, calculée relativement à la solution prête.

6. Un procédé selon la revendication 5, caractérisé en ce que le saccharide est le saccharose.

7. Un procédé selon l'une quelconque des revendications 1 à 6, caractérisé en ce que l'on effectue le traitement thermique en présence de CaCl₂ en une quantité ne dépassant pas 150 mmol/g de solution prête.
